# EUROPÄISCHE PATENTSCHRIFT

(11) **EP 0 357 998 B1**
(45) Veröffentlichungstag und Bekanntmachung des Hinweises auf die Patenterteilung: **07.12.1994**
(21) Anmeldenummer: 89114954.4
(22) Anmeldetag: 12.08.1989
(51) Int. Cl.: C12M 1/26

(54) **Verfahren für eine sterile Handhabung von strömungsfähigen Fermenterproben sowie Vorrichtung zur Durchführung des Verfahrens.**
Process and apparatus to keep liquid fermenter samples sterile
Procédé et appareil pour maintenir stérile des prélèvements liquides de fermentateur

(30) Priorität: 18.08.1988 DE 3828004
(43) Veröffentlichungstag der Anmeldung: 14.03.1990
(73) Patentinhaber: KABLAU HOCHDRUCK-ARMATUREN MENTE GESELLSCHAFT M.B.H., D-21035 Hamburg (DE)
(72) Erfinder: Mente, Edgar Dipl.-Ing., D-2051 Escheburg (DE)
(74) Vertreter: Patentanwälte Hauck, Graalfs, Wehnert, Döring, Siemons

(56) Entgegenhaltungen:
- WO-A-85/03773
- US-A- 2 566 306

## Beschreibung

Die Erfindung bezieht sich auf eine Vorrichtung zur Entnahme von Proben aus Fermentern nach dem Oberbegriff des Patentanspruchs 1.

In Fermentern werden bekanntlich biologische Kulturen gezüchtet. Zur Probenentnahme und zur Impfung des Inhaltes im Fermenter sind strömungsfähige Substanzen aus den Fermentern heraus und in diese hinein zu befördern. Diese Substanzen sind vielfach außerordentlich empfindlich. Die Handhabung muß naturgemäß unter sterilen Bedingungen erfolgen.

Aus der WO 85/03773 ist eine Vorrichtung der eingangs genannten Art bekanntgeworden, bei der ein Probenentnahmerohr von einem mit dem Gehäuse des Fermenters verbundenen Gehäuse abgedichtet umgeben ist. Das Probenentnahmerohr weist ein geschlossenes Ende auf, das ständig über eine O-Ringdichtung in das Innere des Fermenters weist. Das Ansatzgehäuse ist über ein Rohrstück mit einem Anschlußgehäuse für eine Probenflasche verbunden, durch welches Rohrstück eine Verbindungsleitung für eine Injektionsnadel geführt ist, welche das Septum für die Probenflasche durchstechen kann. Injektionsnadel und Entnahmerohr sind beweglich in den ihnen zugeordneten Gehäusen geführt, so daß bei einem Aufeinanderzubewegen beider Gehäuse zunächst die Injektionsnadel das Septum der Probenflasche durchsticht und bei einem weiteren Vorschub das Entnahmerohr weiter in den Fermenter hineingeschoben wird, so daß eine seitliche Bohrung im Probenentnahmerohr im Abstand von seinem Ende in das Innere des Fermenters gelangt. Beide Gehäuse sind mit einem Anschluß für ein Sterilisiermedium verbunden, so daß das Äußere und das Innere des Probenentnahmerohrs sowie das Äußere und das Innere der Injektionsnadel mit Sterilisiermedium gespült werden können, bevor der beschriebene Probenentnahmevorgang vonstatten geht. Ebenso kann eine Sterilisierung nach dem Probenentnahmevorgang durchgeführt werden.

Der Erfindung liegt die Aufgabe zugrunde, eine Vorrichtung zur Entnahme von Proben aus Fermentern zu schaffen, welche sicherstellt, daß alle für die Probenentnahme mit den Kulturen in Berührung gelangenden Teile wirksam sterilisiert werden, ohne daß der Inhalt des Fermenters eine keimabtötende Behandlung erfährt.

Diese Aufgabe wird erfindungsgemäß durch die Merkmale des Patentanspruchs 1 gelöst.

Bei der erfindungsgemäßen Vorrichtung ist das Entnahmerohr als Injektionsnadel ausgebildet und in der Lage, die durch ein Septum abgeschlossene Öffnung des Fermenterbehälters zu durchstechen. Vor und nach der Probenentnahme befindet sich die Injektionsnadel vollständig im Ansatzgehäuse außerhalb des Septums.

Bei der erfindungsgemäßen Vorrichtung befindet sich die Injektionsnadel vor dem Septum des Fermenters und wird erst nach der Sterilisation durch das Septum gestoßen. Erfindungswesentlich ist, daß die Einstichnadel von innen und außen mit Dampf sterilisiert wird, der Inhalt des Fermenters aber keine keimabtötende Behandlung erfährt. Auch nach der Probenentnahme kann wirksam sterilisiert werden, so daß das Gerät im keimfreien Zustand verlassen werden kann.

Bei der Erfindung ist ferner die Bildung von Bakterienstämmen an Dichtringen oder dergleichen ausgeschlossen. Nach Ausgestaltungen der Erfindung sind die Injektionsnadeln von einem Faltenbalg umgeben, welcher Temperaturdehnungen kompensiert. Als Sterilisiermedium wird vorzugsweise Heißdampf verwendet.

Nach einer weiteren Ausgestaltung der Erfindung ist an den Heißdampfanschluß eine Strahldüse angeschlossen, die in einem mit der zweiten Injektionsnadel verbundenen Zwischenbehälter über eine Verbindung einen Unterdruck erzeugt. Die zweite Injektionsnadel mündet am Boden des Zwischenbehälters, und die erste Injektionsnadel ist eine Doppelnadel, von der eine mit dem Boden des Zwischenbehälters und die andere mit dem oberen Teil des Zwischenbehälters verbunden ist. Dadurch kann wirksam eine Probenentnahme vorgenommen werden, auch wenn im Fermenter kein Überdruck herrscht. Die Doppelnadel ermöglicht zugleich ein einfacher Befüllen der Probenflasche, da die eine Nadel die Probenflasche entlüftet. Die Doppelnadel hat ferner den Vorteil, daß die Probenflasche vor der Befüllung mit Heißdampf sterilisiert werden kann.

Vorteilhafte Ausgestaltungen der Erfindung sind in weiteren Unteransprüchen angegeben.

Die Erfindung wird im folgenden anhand von Ausführungsbeispielen erläutert, die in der Zeichnung dargestellt sind. Die Zeichnungen zeigen schematische Darstellungen mit den wesentlichen Teilen, wobei Stütz- oder Führungselemente, wie sie zur Erleichterung der Handhabung vorgesehen sind, herausgelassen sind. Es zeigen:
- Fig. 1a u. 1b: eine schematische Seitenansicht im Schnitt eines Gerätes zur Probenentnahme aus einem Fermenter in Teildarstellung, wobei die Fig. 1a den linken Teil und die Fig. 1b den rechten Teil bezüglich einer strichpunktierten eingezeichneten Trennungslinie darstellen;
- Fig. 2a u. 2b: eine der Fig. 1 entsprechende Darstellung, jedoch eines Gerätes zur Einführung einer Substanz in einen Fermenter, wobei die Fig. 2a hinsichtlich der Zeichnungsdarstellung den rechten Teil und die Fig. 2b den linken Teil des Gerätes zeigt, das in der Anwendung jedoch um 90 ° gedreht ist, derart, daß die Teile der Fig. 2a oben und die Teile der Fig. 2b unten sind;
- Fig. 3: ein Schnitt längs der Linie III-III durch Fig. 2b.

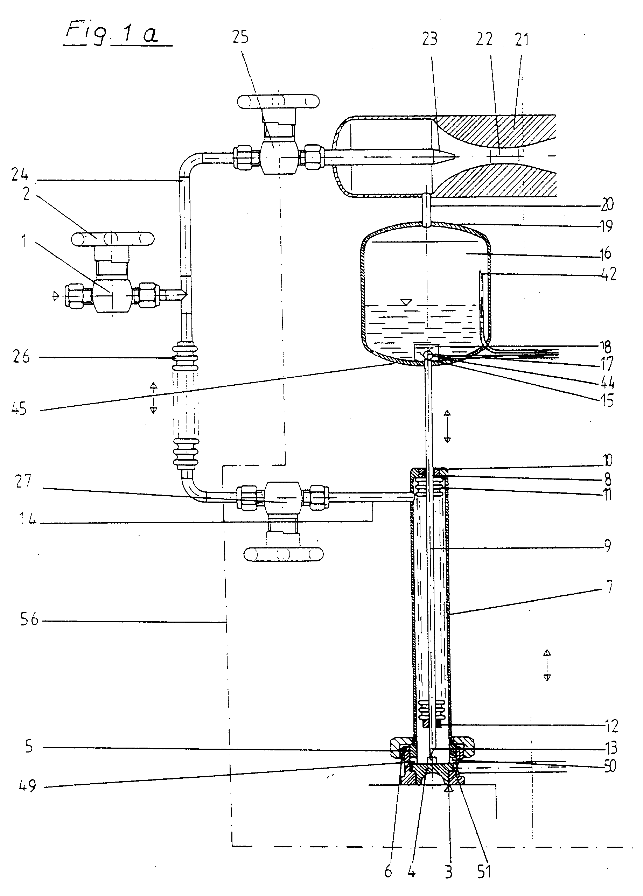

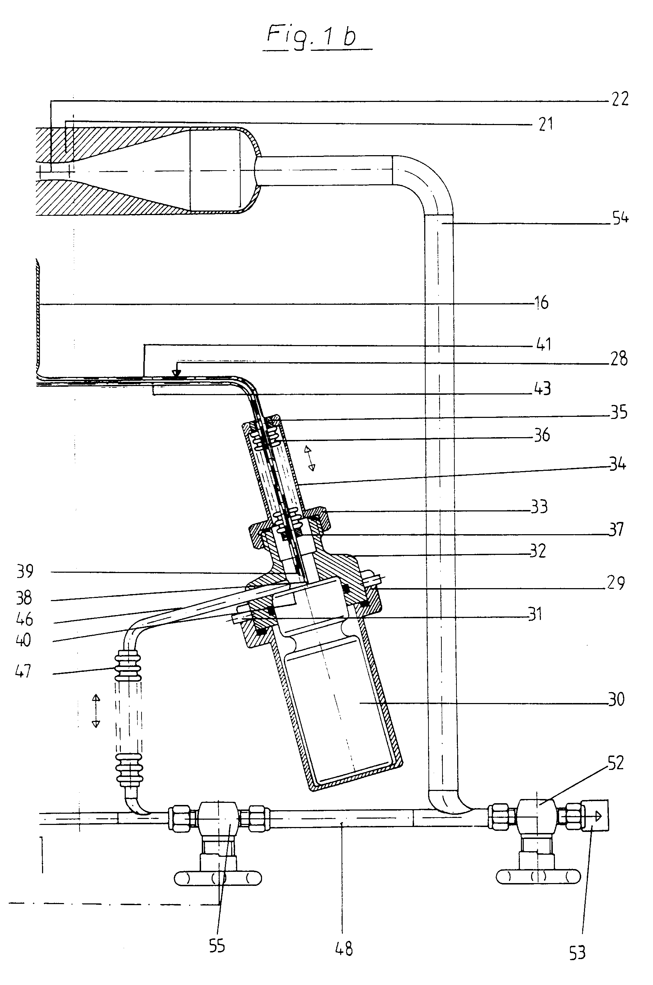

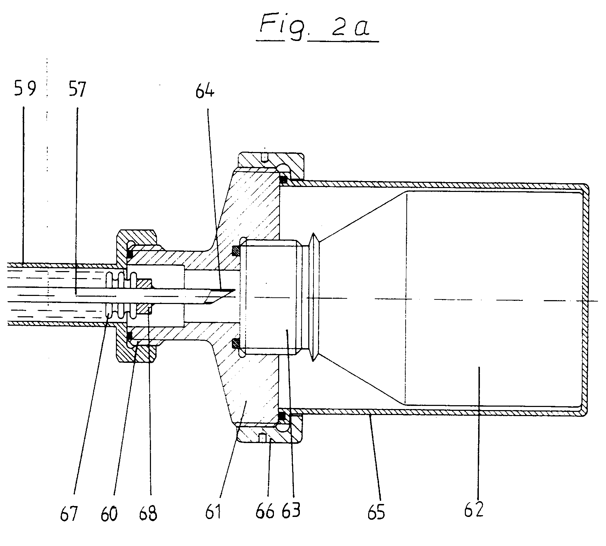

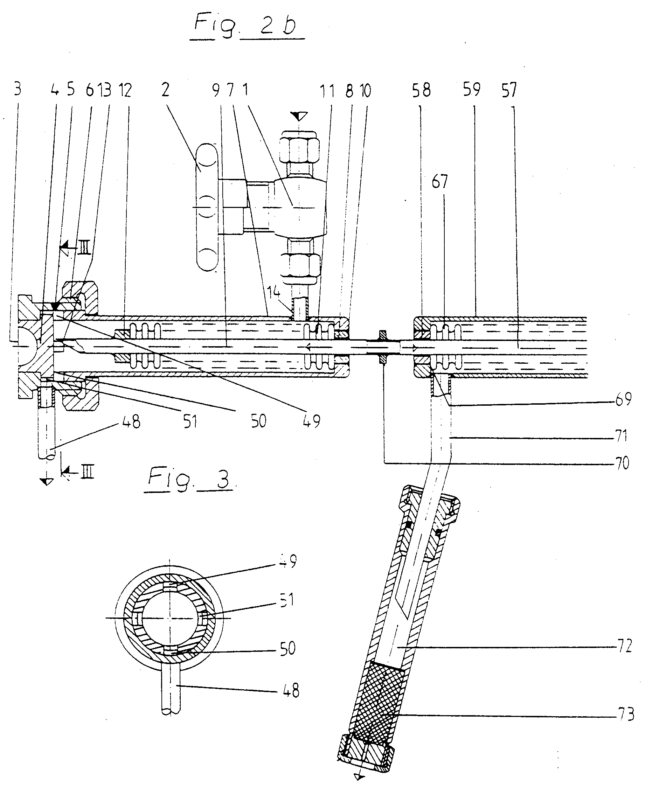

Die Geräte nach den Figuren 1 und 2 haben einen Heißdampfeinlaß 1 in Form einer Armatur mit einem Handrad 2, durch welches der Einlaß gesteuert werden kann.

Beide Geräte sind Aufsatzgeräte am Deckel 3 eines sonst nicht dargestellten, an sich bekannten Fermenters. An diesem Deckel ist ein Septum 4 angeordnet, welches von einer Injektionsnadel durchstochen werden kann, um Zugänge zum Fermenter zu schaffen, während es sich hermetisch abschließt, wenn die Injektionsnadel zurückgezogen wird.

Der Verschluß 5 mit dem Septum 4 ist jeweils als Stutzen auf dem Fermenterdeckel angeordnet und mittels einer Schraubverbindung 6 ist ein langgestrecktes, zylindrisches Gehäuse 7 gasdicht und steril angeflanscht. Hierzu werden bekannte Mittel verwendet. Das zylindrische Gehäuse 7 hat jeweils an seinem oberen, vom Fermenterdeckel 3 abgekehrten Ende einen auch Führungsaufgaben erfüllenden Durchgang 8 für eine Injektionsnadel 9. An dem Rand des Durchgangs ist innen steril abgedichtet ein Ende 10 eines Faltenbalges 11 angeordnet, der sich in das Gehäuse 7 erstreckt und in der Nähe des unteren Endes des Gehäuses über eine sterile hermetische Abdichtung 12 mit der Injektionsnadel 9 verbunden ist. Der Faltenbalg 11 besteht aus Edelmetall. Er hält die Injektionsnadel mit einer Spitze 13 über dem Septum 4. In das Gehäuse 7 mündet zweckmäßig im oberen Bereich eine Zuführungsleitung 14, die zum Heißdampfeinlaß führt. Eintretender Heißdampf bespült daher den Faltenbalg 11 von außen und gelangt über die Öffnung in der Spitze 13 in die Injektionsnadel 9, wobei in diesem Bereich auch das Septum 4 sterilisiert wird, und dann durch die Injektionsnadel in einen angeschlossenen Behälter.

Zunächst wird auf die Fig. 1 Bezug genommen. Die aus dem Gehäuse 7 herausgeführte Injektionsnadel 9 mündet mit ihrem oberen Ende 15 im Boden eines Zwischenbehälters 16. An der Mündung ist ein Rückschlagventil mit einem Rückschlagventilkörper 17 vorgesehen, der in einem durchlässigen Käfig 18 innerhalb des Zwischenbehälters angeordnet ist. Der Zwischenbehälter ist insofern starr mit der Injektionsnadel verbunden. Der Zwischenbehälter 16 steht durch die obere Wand 19 über eine Verbindungsleitung 20 mit einer Strahldüse 21 in Verbindung. Diese Verbindungsleitung 20 mündet an einer Stelle der Strahldüse, an welcher im Betrieb Unterdruck auftritt, d.h.vor der Düsenverengung 22. In diese ist eine Dampfzuführungsdüse 23 gerichtet, die abgedichtet in das Gehäuse der Strahldüse 21 eingeführt ist und über eine beispielsweise starre Leitung 24 mit dem Heißdampfeinlaß 1 bzw. seiner Armatur in Verbindung steht. In der Leitung 24 ist vor der Dampfzuführungsdüse eine Armatur 25 angeordnet, welche die Leitung 24 öffnen oder sperren kann.

Es ergibt sich somit eine starre Baugruppe aus einer Strahldüse 21, dem Zwischenbehälter 16 und der Injektionsnadel 9.

Wenn diese Injektionsnadel 9 nun zum Durchstechen des Septums 4 nach unten gedrückt wird, muß die gesamte Baugruppe bewegt werden, wobei das Gehäuse der Strahldüse 21 als Handgriff dienen kann.

Die Zuführungsleitung 14 vom Heißdampfeinlaß 1 zum Gehäuse 7 besitzt an einem Abschnitt parallel zur Achsrichtung der Nadel 9 einen Faltenbalg 26, der diese Bewegungen gegenüber dem Gehäuse 7 aufnimmt. In der Zuführungsleitung 14 ist eine Sperrarmatur 27 angeordnet.

Der Zwischenbehälter 16 steht über eine sogenannte Doppelnadel 28, welche in der Fig. 1 als solche gezeichnet ist, aber auch von einem versteifenden Gehäuse umgeben sein kann, mit einer Aufnahme 29 für eine Probenflasche 30 in Verbindung. Diese Aufnahme ist durch einen Bajonettverschluß 31 mit einem Kopfstück 32 verbunden, das durch eine Schraubarmatur 33 mit einem zylindrischen Gehäuse 34 in Verbindung steht, in welches die Doppelnadel 28 eingeführt ist. Das zylindrische Gehäuse hat einen beweglichen Durchgang 35 für die Doppelnadel. An diesem Durchgang ist innen ein Faltenbalg 36 angeordnet, dessen anderes Ende an einem Nadelkopf 37 im Kopfstück 32 steril abgedichtet an der Doppelnadel angeschlossen ist. Aus dem Kopfstück ragen die beiden Enden 38, 39 der Doppelnadel so weit hervor, daß sie das Septum am Verschluß der Probenflasche 30 durchstoßen können. Dieser Verschluß ist mit 40 bezeichnet. Das eine Ende der Doppelnadel steht gegenüber dem anderen Ende hervor. Dieses eine Ende gehört zu einem Doppelnadelteil 41, welcher innerhalb des Zwischenbehälters nach oben geführt ist und bei 42 im oberen Bereich mündet.

Dieser Doppelnadelteil dient der Entlüftung. Der andere Doppelnadelteil 43 mit dem Ende 39 hat eine Mündung 44 über dem Boden 45 des Zwischenbehälters und dient zum Durchgang der Probe aus dem Zwischenbehälter in die Probenflasche 30.

Wie aus Fig. 1 ersichtlich ist,sind die Probenflasche 30 und ihre Aufnahme 29 sowie das Kopfstück 32 und das zylindrische Gehäuse 34 schräg zur Vertikalen angeordnet, die durch die Erstreckung der Nadel 9 bestimmt ist. Dadurch ergibt sich die Möglichkeit, in das Kopfstück 32 eine Abführungsleitung 46 einzuführen, die über einen Faltenbalg 47 mit einer Heißdampf- oder Kondensatableitung 48 in Verbindung steht, die an dem unteren Teil des Gehäuses 7 auf dem Septum 4 angeschlossen sind.

Das Gehäuse 7 hat an seinem Fuß Durchgänge 49, 50, die in einen Aufnahmeraum 51 münden, an welchem die Heißdampf- oder Kondensatableitung angeschlossen ist. Diese steht über eine Armatur 52 mit einer nicht dargestellten, im ganzen mit 53 bezeichneten Entsorgung über ein nicht dargestellten Mikrofilter in Verbindung. Die Armatur ist eine Regel- oder Absperrarmatur. Vor ihr mündet eine vom Ausgang der Strahldüse 21 führende Leitung 54 in die Heißdampf- oder Kondensatableitung.

Gemäß Darstellung ist die Leitung 54 flexibel, so daß sie bei der Höhenbewegung der Baugruppe mit der Strahldüse 21 nachgeben kann. Bei starrer Ausführung würden ihre Teile durch einen Faltenbalg verbunden sein.

Hinsichtlich des Betriebes ist es zweckmäßig, daß der Querschnitt der Leitung 54 größer ist als beispielsweise der Querschnitt der starren Leitung 24.

Von Bedeutung ist, daß in der Heißdampf- oder Kondensatableitung 48 zwischen dem Anschluß der Abführungsleitung 46 und der Leitung 54 eine Sperrarmatur 55 angeordnet ist. Diese ist zweckmäßig entsprechend der gestrichelt eingezeichneten Funktionsverbindung 56 mit der Armatur 25 vor der Strahldüse 21 gekoppelt derart, daß bei Öffnung der einen Armatur die andere geschlossen ist.

Wenn mit dem Gerät nach Fig. 1 eine Probe entnommen werden soll, wird dieses Gerät zunächst mit Heißdampf gespült und sterilisiert. Der Heißdampf tritt bei geschlossener Armatur 25 in das Gehäuse 7 ein, umströmt den Faltenbalg 11, gelangt durch die Nadel 9 in den Zwischenbehälter 16, von diesem einerseits in die Strahldüse 21 und andererseits durch die Doppelnadel in das Gehäuse 34 bzw. das Kopfstück 32, so daß auch der Verschluß 40 mit seinem Septum sterilisiert wird. Aus diesem Raum erfolgt die Abströmung unter gleichzeitiger Kondensatabführung durch die Abführungsleitung 46 zur Entsorgung 53. Entsprechend strömt Heißdampf durch die Leitung 54 ab.

Wenn die Strahldüse 21 besonders sterilisiert werden soll, wird die Armatur 27 geschlossen und 25 geöffnet, so daß dann im Bereich der Strahldüse 21 eine intensive Heißdampfdurchströmung erfolgt. Das Kondensat strömt durch die beschriebene Kondensatablässe ab.

Im Betrieb wird dann, wenn das Septum 4 durchstoßen ist und in der Strahldüse 21 ein Unterdruck erzeugt wird, der Kondensatablaß durch Sperrung der Armatur 55 abgeschlossen. Gleichzeitig wird, da eine Sterilisierung vorher stattgefunden hat, die Armatur 27 geschlossen.

Der sich im Zwischenbehälter 16 einstellende Unterdruck saugt aus dem Fermenter die gewünschte Probe an. Nachdem die Aufnahme 29 mit der Probenflasche 30 so vorgeschoben ist, daß die Doppelnadel den Verschluß durchstößt, fließt die Probe in die Probenflasche 30. Nach deren Freigabe und Rückführung in die Ausgangsstellung unter Entspannung des Faltenbalges schließt sich das Septum des Verschlusses 40.

Jetzt kann nach Abschluß der Armatur 25 eine Durchspülung und Sterilisation in der beschriebenen Weise erfolgen, um auch den Rest der Probe aus dem Zwischenbehälter unter gleichzeitiger Sterilisierung auszutreiben.

Fig. 2 zeigt ein Gerät zur Impfung des Inhaltes des Fermenters. In dieser Ausführung ist die aus dem Gehäuse 7 herausgeführte Injektionsnadel mit ihrem oberen Abschnitt 57 durch eine Durchgangs- und Führungsöffnung 58 in ein entsprechendes zylindrisches Gehäuse 59 eingeführt. Auf diesem zylindrischen Gehäuse ist mittels eines Schraub- oder Bajonettverschlusses 60 ein Trägerkopf 61 für einen Vorratsbehälter 62 angeordnet, dessen Inhalt in den Fermenter, gegebenenfalls unter Dosierung einzuführen ist. Der Vorratsbehälter ist mit seinem Verschluß 63 nach unten gerichtet im Trägerkopf 61 abgedichtet angeordnet. Dieser Verschluß hat ein durchstoßbares Septum und liegt über dem oberen Ende 64 der Nadel 9 bzw. des oberen Nadelabschnittes 57. Der Vorratsbehälter 62 kann mit seiner Aufnahme 65 über einen Bajonettverschluß 66 oder einen entsprechenden Verschluß im Trägerkopf 61 auswechselbar angeordnet sein.

Der obere Abschnitt 57 der Injektionsnadel 9 ist innerhalb des Gehäuses in einem Faltenbalg 67 geführt, dessen inneres Ende bei 68 abgedichtet mit der Injektionsnadel verbunden ist und dessen unteres Ende 69 an den Rand der Durchgangs-und Führungsöffnung 58 steril angeschlossen ist. Insofern ist das obere Ende 64 mit der Mündung der Injektionsnadel von der Umgebung abgeschlossen.

Die Faltenbälge 11 und 67 halten die Anordnung des Gerätes im gezeigten Zustand. Zwischen den Gehäusen 7 und 59 ist an der Injektionsnadel 9 ein flanschartiger Vorsprung 70 angeordnet, der mit den Durchgangsrändern der Gehäuse 7 und 59 in Anlage kommen kann.

Wenn die Aufnahme 65 als Handgriff verwendet und nach unten gedrückt wird, dann wird nach Maßgabe der Flexibilität der Faltenbälge auch die Nadel 9 nach unten bewegt. Wenn der Rand der Durchgangs- und Führungsöffnung 58 den flanschartigen Vorsprung 70 erreicht, wird die Nadel 9 unter Ausdehnung des Faltenbalges 11 nach unten gedrückt, so daß das Septum 4 durchstoßen wird. Wenn der flanschartige Vorsprung 70 den Rand des Durchganges 8 erreicht und sich dort abstützt, erzwingt eine Weiterbewegung der Aufnahme 65 den Durchstich des Endes 64 der Injektionsnadel 9 durch das Septum des Verschlusses 63, so daß dann die Verbindung zwischen dem Vorratsbehälter 62 und dem Fermenter hergestellt ist.

Das zylindrische Gehäuse 59 ist am unteren Ende mit einer Ableitung 71 versehen, die in ein Abführungsgehäuse 72 mit einem Mikrofilter 73 führt, der in einer nicht dargestellten Weise an eine Versorgung angeschlossen ist.

In der oben anhand der Fig. 1 beschriebenen Weise kann eine Spülung und Sterilisation durch Heißdampfeinlaß bei 14 stattfinden, wobei die Injektionsnadel den Dampf auch an den Verschluß 63 führt, und der Faltenbalg 67 außen umströmt wird. Heißdampf und Kondensat können durch 71 austreten. Im Gehäuse 7 anfallendes Kondensat tritt durch die Heißdampf-und Kondensatableitung 48 aus, die in mehr Einzelheiten anhand der Fig. 1 beschrieben ist.

In der vorteilhaften Ausführung der erfindungsgemäßen Ausgestaltung werden bei der Sterilisation alle Teile, die mit Substanzen in Berührung kommen können, von Dampf durch- bzw. umströmt, und es gibt wegen der elektro-chemisch innen polierten Flächen keine Oberfläche mehr, die einen Ansatz für Kulturen bilden könnte. Das Gerät ist in der bevorzugten Ausführungsform eine reine Metallkonstruktion, wozu insbesondere die Faltenbälge beitragen, die an den Teilen, die sterilisiert werden, keiner Dichtung bedürfen. Die Armaturen sind insbesondere als totraumfreie PTFE-beschichtete Zylinderkükenhähne ausgeführt.

Wie anhand Fig. 1 gezeigt ist, hat das Gehäuse 7 an seinem Fuß die Durchgänge 49, 50, die an einem Aufnahmeraum 51 um das Septum 4 münden, an welchen Aufnahmeraum die Leitung 48 angeschlossen ist.

Fig. 3 verdeutlicht diese Ausführung. Es ist erkennbar, daß entsprechend den Durchgängen 49, 50 zwei weitere Durchgänge um 90 ° versetzt angeordnet sind, wobei im Bereich der Durchgänge Abschnitte des Aufnahmeraums 51 erkennbar sind.

## Patentansprüche

1. Vorrichtung zur Entnahme von Proben aus Fermentern, mit einem fest mit dem Behälter (3) des Fermenters verbindbaren Ansatzgehäuse (7), einem am entgegengesetzten Ende in das Gehäuse (7) einführbaren beweglich gelagerten Probenentnahmerohr (9), das mit seinem vorderen Ende (13) über eine Öffnung in den Behälter (3) einführbar ist, einem Halter (29, 32, 61, 65) für eine Probenflasche (30, 62), die durch ein Septum (40) abgeschlossen ist, einer in einem abgedichteten Anschlußgehäuse (34, 59) des Probenhalters abgedichtet und beweglich gehaltenen Injektionsnadel (41, 43, 57), die mit dem Entnahmerohr (9) verbunden und über das Septum (40) in die Probenflasche (30, 62) einführbar ist, einem Anschluß am Ansatzgehäuse (7) für die Zufuhr und einem Anschluß am Anschlußgehäuse (34, 59) für die Abfuhr eines Sterilisiermediums, wobei der das Entnahmerohr (9) umgebende Raum, das Innere des Entnahmerohrs (9) und der Injektionsnadel (41, 43 57) sowie das Äußere der Injektionsnadel (41, 43, 57) mit Sterilisiermedium in Kontakt bringbar sind, dadurch gekennzeichnet, daß das Entnahmerohr als Injektionsnadel (9) ausgebildet ist, die Öffnung im Behälter (3) durch ein Septum (4) abgeschlossen ist und die Injektionsnadel (9) vor und nach der Probenentnahme sich vollständig im Ansatzgehäuse (7) außerhalb des Septums (4) befindet.

2. Vorrichtung nach Anspruch 1, dadurch gekennzeichnet,daß die Injektionsnadel (9) im Ansatzgehäuse (7) abgedichtet von einem Faltenbalg (11) umgeben ist.

3. Vorrichtung nach Anspruch 1 oder 2, dadurch gekennzeichnet, daß die Injektionsnadel (41, 43, 57) im Anschlußgehäuse des Probenflaschenhalters ebenfalls abgedichtet von einem Faltenbalg (36, 67) umgeben ist.

4. Vorrichtung nach einem der Ansprüche 1 bis 3, dadurch gekennzeichnet, daß als Sterilisiermedium Heißdampf dient.

5. Vorrichtung nach Anspruch 1 und 4, dadurch gekennzeichnet, daß an den Heißdampfanschluß eine Strahldüse (21) angeschlossen ist, die in einem mit der zweiten Injektionsnadel (9) verbundenen Zwischenbehälter (16) über eine Verbindungsleitung (20) einen Unterdruck erzeugt, wobei das am Boden des Zwischenbehälters (16) mündende Ende der zweiten Injektionsnadel (9) mit einem Rückschlagventil (17, 18) im Sinne des Abschlusses der Nadel (9) versehen ist und der Zwischenbehälter (16) über eine Doppelnadel (28) mit dem Anschlußgehäuse in Verbindung steht, von denen eine Nadel (43) zur Probenzuführung am Boden des Zwischenbehälters (16) und die andere Nadel (41) zur Entlüftung im oberen Teil des Zwischenbehälters (16) münden.

6. Vorrichtung nach Anspruch 5, dadurch gekennzeichnet,daß ein Kopf der Doppelnadel (37) über der herausnehmbar angeordneten Probenflasche (30) an einem Faltenbalg (36) vorgesehen ist.

7. Vorrichtung nach einem der Ansprüche 4 bis 6, dadurch gekennzeichnet, daß eine Heißdampfzuführung (14) mit Faltenbalg (26) mit dem Ansatzgehäuse (7) verbunden ist.

8. Vorrichtung nach einem der Ansprüche 4 bis 7, dadurch gekennzeichnet, daß eine Heißdampf- und Kondensatwasserabführung (49 bis 51, 53; 69, 71) an der tiefsten Stelle des Ansatzgehäuses (7) vorgesehen ist.

9. Vorrichtung nach einem der Ansprüche 4 bis 8, dadurch gekennzeichnet, daß über dem Septum (40) im Anschlußgehäuse eine Kondensatwasser- und Heißdampfabführung (46, 48, 53) vorgesehen ist.

10. Vorrichtung nach einem der Ansprüche 4 bis 9, dadurch gekennzeichnet, daß Absperrarmaturen (25, 55) vor der Strahldüse und einer Kondensatableitung (48) angeordnet und wechselweise gesteuert sind derart, daß beim Öffnen einer Absperrarmatur die andere geschlossen ist und umgekehrt und daß die Dampf- und Kondensatabführung über ein Mikrofilter (73) an eine Entsorgung (53) angeschlossen ist.

11. Vorrichtung nach einem der Ansprüche 3 bis 10, dadurch gekennzeichnet, daß das Anschlußgehäuse (34, 59) unter Stauchung des Faltenbalges (36, 67) in Richtung der Injektionsnadel bewegbar ist.

12. Vorrichtung nach einem der Ansprüche 5 bis 11, dadurch gekennzeichnet, daß die zweite Injektionsnadel (9), der Zwischenbehälter (16) und die Strahldüse (21) zu einer Baugruppe zusammengefaßt sind, die in bezug auf das Ansatzgehäuse (7) am Fermenter beweglich geführt ist.

13. Vorrichtung nach Anspruch 1, dadurch gekennzeichnet, daß zwischen Ansatzgehäuse (7) und Anschlußgehäuse (59) an der Verbindung zwischen den Injektionsnadeln (9, 57) ein flanschartiger Vorsprung (70) vorgesehen ist, welcher bei Heranbewegung des Endes eines Gehäuses (59) die Injektionsnadel (9) mitnimmt und im anderen Gehäuse (7) vorschiebt.

## Claims

1. Apparatus for withdrawing samples from fermenters, comprising a first casing (7) suited to be rigidly connected to the container (3) of the fermenter, a movably supported sample withdrawing tube (9) to be inserted into the frist casing (7) at the opposite end thereof, the forward end (13) of said tube to be insertable through an opening into the container (3), further comprising a holder (29, 32, 61, 65) for a sampling bottle (30, 62) which is closed by a septum (40), an injection needle (41, 43, 57) which is sealingly and movably held in a sealed second casing (34, 59) of the sample holder, which injection needle is connected to the discharge tube (9) and insertable through the septum (40) into the sampling bottle (30, 62), a connecting means at the first casing (7) for supplying and connecting means at the second casing (34, 59) for discharging a sterilizing medium, wherein the space provided around the withdrawing tube (9), the inner space of the withdrawing tube (9) and of the injection needle (41, 43, 57) as well as the outer space around the injection needle (41, 43, 57) can be brought in contact with sterilizing medium, characterized in that the withdrawing tube is shaped as an injection needle (9), that the opening in the container (3) is closed by a septum (4) and that the injection needle (9) is completely housed in the first casing (7) outside of the septum (4) before and after withdrawing the sample.

2. The apparatus of claim 1, characterized in that the injection needle (9) is sealingly enclosed by a bellows (11) in the first casing (7).

3. The apparatus of claim 1 or 2, characterized in that the injection needle (41, 43, 57) is sealingly enclosed by a bellows (36, 67) in the second casing of the sampling bottle holder.

4. The apparatus of one of claims 1 to 3, characterized in that hot steam is used as sterilizing medium.

5. Apparatus of claim 1 and 4, characterized in that a nozzle (21) is connected to the hot steam connecting means to produce a vacuum pressure in an intermediate container (16) connected through a connecting line (20) to the second injection needle (9), wherein the end of the second injection needle (9) opening at the bottom of the intermediate container (16) is provided with a check valve (17, 18) to close the needle (9) and that the intermediate container (16) communicates with the second casing through a double needle (28), wherein one needle (43) thereof opens at the bottom of the intermediate container (16) for withdrawing a sample and wherein the other needle (41) opens into the upper portion of the intermediate container (16) for venting.

6. Apparatus of claim 5, characterized in that a head of the double needle (37) above the removably mounted sampling bottle (30) is provided at a bellows.

7. Apparatus of one of claims 4 to 6, characterized in that a hot steam supply (14) with bellows (26) is connected to the first casing (7).

8. Apparatus of one of claims 4 to 7, characterized in that a hot steam and condensator discharge line (49 to 51, 53; 69, 71) is provided at the lowermost portion of the first casing (7).

9. Apparatus of one of claims 4 to 8, characterized in that a condensator water and hot steam discharge line (46, 48, 53) is provided above the septum (40) in the second casing.

10. Apparatus of one of claims 4 to 9, characterized in that closing means (25, 55) upstream of the nozzle and a condensate discharge line (48) are provided which closing means are controlled such with respect to each other that in opening one of the closing means the other closing means is closed and vice versa, in that the steam and condensate discharge line is connected through a microfilter (73) to a discharge means (53).

11. The apparatus of one of claims 3 to 10, characterized in that the second casing (34, 59) is movable along the direction of the injection needle while upsetting the bellows.

12. Apparatus of one of claims 5 to 11, characterized in that the second injection needle (9), the intermediate container (16) and the nozzle (21) are combined to form a module which is movably guided with respect to the first casing (7) attached to the fermenter.

13. Apparatus of claim 1, characterized in that a flange like projection (70) is provided between the first casing (7) and the second casing (59) at the joining portion between the injection needles (9, 57) which projection drivingly engages the injection needle (9) when the end of a casing (59) approaches to move the injection needle forwardly in the other casing (7).

## Revendications

1. Dispositif destiné à prélever des échantillons dans des fermenteurs, comportant :
un carter rapporté (7) pouvant être relié de manière fixe au réservoir (3) du fermenteur,
un tube de prélèvement d'échantillon (9), qui peut être introduit à l'extrémité opposée dans le carter (7), et qui est monté de manière mobile et peut être introduit par son extrémité avant (13) dans le réservoir (3) par l'intermédiaire d'une ouverture,
un support (29, 32, 61, 65) pour une bouteille d'échantillon (30, 62) qui est formée par un septum (40), une aiguille d'injection (41, 43, 57) qui est maintenue de manière mobile et étanche dans un carter de raccordemènt (34, 59) étanche du support d'échantillon, et qui est reliée au tube de prélèvement (9) et peut être introduite dans la bouteille d'échantillon (30, 62) par l'intermédiaire du septum,
un raccord sur le carter rapporté (7) pour l'introduction d'un fluide de stérilisation, et un raccord sur le carter de raccordement (34, 59) pour l'évacuation de ce fluide de stérilisation,
l'espace entourant le tube de prélèvement (9), et l'intérieur du tube de prélèvement. (9) et de l'aiguille d'injection (41, 43, 57), ainsi que l'extérieur de l'aiguille d'injection (41, 43, 57) pouvant être amenés en contact avec du fluide de stérilisation,
caractérisé en ce que le tube de prélèvement est réalisé sous la forme d'une aiguille d'injection (9), en ce que l'ouverture dans le réservoir (3) est obturée par un septum (4), et en ce que l'aiguille d'injection (9), avant et après le prélèvement de l'échantillon, se trouve totalement. dans le carter rapporté (7), en-dehors du septum (4).

2. Dispositif selon la revendication 1, caractérisé en ce que l'aiguille d'injection (9) est entourée de manière étanche par un soufflet (11), dans le carter rapporté (7).

3. Dispositif selon la revendication 1 ou 2, caractérisé en ce que l'aiguille d'injection (41, 43, 57) est également entourée de manière étanche par un soufflet (36, 67) dans le carter de raccordement du support de bouteille d'échantillon.

4. Dispositif selon l'une des revendications 1 à 3, caractérisé en ce que l'on utilise de la vapeur surchauffée on guise de fluide de stérilisation.

5. Dispositif selon les revendications 1 et 4, caractérisé en ce qu'au raccord de vapeur surchauffée est raccordé un éjecteur (21), qui produit, par l'intemédiaire d'une conduite de liaison (20), une dépression dans un réservoir intermédiaire (16) relié à la seconde aiguille d'injection (9), l'extrémité de la seconde aiguille d'injection (9), qui débouche dans le fond du réservoir intermédiaire (16), étant munie d'un clapet anti-retour (17, 18) agissant dans le sons d'une obturation de l'aiguille (9), et le réservoir intermédiaire (16) étant relié au carter de raccordement par l'intermédiaire d'une aiguille double (37), dont une aiguille (43), destinée à l'amenée de l'échantillon, débouche dans le fond du réservoir intermédiaire (16), et l'autre aiguille (41) débouche dans la partie supérieure du réservoir intermédiaire (16), pour la purge.

6. Dispositif selon la revendication 5, caractérisé en ce qu'une tête de l'aiguille double (37) est prévue sur un soufflet (36), au-dessus de la bouteille d'échantillon (30) amovible.

7. Dispositif selon l'une des revendications 4 à 6, caractérisé en ce qu'une alimentation de vapeur surchauffée (14) pourvu d'un soufflet (26), est reliée au carter rapporté (7).

8. Dispositif selon l'une des revendications 4 à 7, caractérisé en ce qu'une évacuation de vapeur surchauffée et de condensats d'eau (49 à 51, 53; 69, 71) est prévue au niveau le plus bas du carter rapporté (7).

9. Dispositif selon l'une des revendications 4 à 8, caractérisé en ce qu'au-dessus du septum (40) dans le carter de raccordement, est prévue une évacuation de condensats d'eau et de vapeur surchauffée (46, 48, 53),

10. Dispositif selon l'une des revendications 4 à 9, caractérisé en ce que des vannes d'isolement (25, 55) sont disposées en amont de l'éjecteur (21) et d'une conduite d'évacuation des condensats (48), et sont commandées alternativement de manière telle que, lors de l'ouverture de l'une des vannes d'isolement, l'autre soit fermée et inversement, et en ce que l'évacuation de la vapeur et des condensats est raccordée, par l'intermédiaire d'un microfiltre (73), à une sortie de collecte (53).

11. Dispositif selon l'une des revendications 3 à 10, caractérisé en ce que le carter de raccordement (34, 59) peut être déplacé en direction de l'aiguille d'injection, en comprimant le soufflet (36, 67).

12. Dispositif selon l'une des revendications 5 à 11, caractérisé en ce que la seconde aiguille d'injection (9), le réservoir intermédiaire (16) et l'éjecteur (21) sont regroupés en un module, qui est guidé sur le fermenteur, de manière mobile par rapport au carter rapporté (7).

13. Dispositif selon la revendication 1, caractérisé en ce qu'entre le carter rapporté (7) et le carter de raccordement (59), au niveau de la liaison entre les aiguilles d'injection (9, 57), est prévue une saillie en forme de bride (70), qui lors du rapprochement de l'extrémité d'un carter (59), entraîne l'aiguille d'injection (9) et la fait avancer dans l'autre carter (7).
